# EUROPÄISCHE PATENTSCHRIFT

(11) **EP 1 890 261 B1**
(45) Veröffentlichungstag und Bekanntmachung des Hinweises auf die Patenterteilung: **18.02.2009**
(21) Anmeldenummer: 06016945.5
(22) Anmeldetag: 14.08.2006
(51) Int. Cl.: G06T 7/00

(54) **Registrierung von MR-Daten anhand generischer Modelle**
Registration of MR data using generic models
Recalage des données d'imagerie par résonance magnétique en utilisant des modèles génériques

(43) Veröffentlichungstag der Anmeldung: 20.02.2008
(73) Patentinhaber: BrainLAB AG, 85622 Feldkirchen (DE)
(72) Erfinder: Feilkas, Thomas, 85567 Grafing (DE); Schaffrath, Claus, 81377 München (DE)
(74) Vertreter: Schwabe - Sandmair - Marx

(56) Entgegenhaltungen:
- EP-A1- 1 348 394
- FLEUTE M ET AL: "NONRIGID 3-D/2-D REGISTRATION OF IMAGES USING STATISTICAL MODELS" LECTURE NOTES IN COMPUTER SCIENCE, SPRINGER VERLAG, BERLIN, DE, 19. September 1999 (1999-09-19), Seiten 138-147, XP008010626 ISSN: 0302-9743
- JIANHUA YAO ET AL: "Assessing accuracy factors in deformable 2d/3d medical image registration using a statistical pelvis model" PROCEEDINGS OF THE EIGHT IEEE INTERNATIONAL CONFERENCE ON COMPUTER VISION. (ICCV). NICE, FRANCE, OCT. 13 - 16, 2003, INTERNATIONAL CONFERENCE ON COMPUTER VISION, LOS ALAMITOS, CA : IEEE COMP. SOC, US, Bd. 2, 13. Oktober 2003 (2003-10-13), Seiten 1329-1334, XP010662546 ISBN: 0-7695-1950-4

## Beschreibung

Die vorliegende Erfindung betrifft ein Verfahren und eine Vorrichtung zur Registrierung eines patientenuncharakteristischen dreidimensionalen Magnetresonanz(MR)-Datensatzes mit patientencharakteristischen Bilddaten, insbesondere mit mindestens zwei Fluoro-Bildern eines Patienten. Basierend auf diesen Bilddaten kann anschließend eine computergestützte, medizinische Navigation durchgeführt werden.

Bei der Untersuchung eines Patienten oder zur Vorbereitung eines chirurgischen Eingriffes, insbesondere bei chirurgischen Eingriffen im Bereich von Knochen, wie zum Beispiel bei einer Wirbelsäulen-, Hüftgelenks- oder Knieoperation, werden Röntgenaufnahmen oder Computertomographie(CT)-Aufnahmen der betroffenen Körperstruktur gemacht, mit welchen Knochenstrukturen gut dargestellt werden können, wobei Röntgenstrahlen verwendet werden, welche eine gesundheitliche Belastung darstellen.

Magnetresonanz- oder Kernspintomographie-Aufnahmen (MR-Aufnahmen), welche ohne gesundheitliche Belastung für einen Patienten erstellt werden können, eignen sich zwar zur Darstellung von weichem Gewebe, jedoch können Knochenstrukturen bei MR-Aufnahmen im Allgemeinen nur schlecht oder gar nicht erkannt werden.

Um sowohl Knochenstrukturen als auch Weichteile gemeinsam darstellen zu können, ist es nötig, dass CT-Aufnahmen oder Röntgenaufnahmen mit MR-Aufnahmen eines Patienten registriert oder fusioniert werden. Hiermit ist jedoch zum einen ein hoher Kostenaufwand verbunden, da Computer-Tomographen und Magnetresonanz-Tomographen sowohl in der Anschaffung als auch in der Wartung und im Betrieb sehr teuer sind. Zum anderen muss eine Vielzahl an CT-Aufnahmen erstellt werden, so dass die Strahlenbelastung für den Patienten sehr hoch ist.

Es ist versucht worden, Systeme zu entwickeln, die sich ohne vorab separat erfasste Körperstrukturdaten des Patienten einsetzen lassen, beispielsweise auf der Basis von generischen Modellen von Bilddatensätzen für Körperstrukturen. Jedoch mangelt es solchen Systemen an der erforderlichen Genauigkeit für den jeweils zu behandelnden Patienten.

Die EP 1348 394 betrifft ein Verfahren zur computergestützten medizinischen Navigation, bei dem mittels einer Positionserfassungseinheit die aktuelle Position eines Patienten oder eines Patientenkörperteils sowie die Positionen von medizinischen Behandlungsgeräten oder behandlungsunterstützenden Geräten erfasst werden, und bei dem die erfassten Positionsdaten Körperstrukturdaten zugeordnet werden, um die Körperstrukturdaten in Zuordnung zu den Positionsdaten gemeinsam im Rahmen der Behandlungsunterstützung zu verwenden, wobei Körperstrukturdaten verwendet werden, die auf der Basis eines dreidimensionalen generischen Modells erhalten werden, wobei das Modell durch eine Datenverknüpfung auf zweidimensionaler Ebene mit patientencharakteristischen, zweidimensionalen Erfassungsdaten angepasst wird.

Diese generischen Modelle basieren zumeist jedoch nicht auf MR-Datensätzen. Damit können generische MR-Daten nicht entsprechend verformt und mit den Fluoro-Bildern registriert werden.

Es ist eine Aufgabe der vorliegenden Erfindung ein Verfahren und eine Vorrichtung bereit zu stellen, welche die Nachteile aus dem Stand der Technik überwinden. Es ist weiter eine Aufgabe der vorliegenden Erfindung ein Verfahren und eine Vorrichtung bereit zu stellen, welche eine kostengünstige, patientenschonende Registrierung von patientenuncharakteristischen MR-Aufnahmen mit patientencharakteristischen Bilddaten mit geringer Strahlenbelastung ermöglichen

Diese Aufgabe wird durch den Gegenstand der unabhängigen Ansprüche gelöst. Vorteilhafte Ausführungsformen ergeben sich aus den abhängigen Ansprüchen.

Bei dem erfindungsgemäße Verfahren zur Registrierung eines patientenuncharakteristischen dreidimensionalen Magnetresonanz(MR)-Datensatzes mit patientencharakteristischen Erfassungsdaten, wie mit Bilddaten mindestens zweier Fluoro-Bilder eines Patienten, wird ein dreidimensionales generisches oder statistisches Modell, insbesondere ein Oberflächenmodell, eines Körpers oder Körperteils erstellt oder bereitgestellt. Zur Erzeugung des generischen Modells können CT-Aufnahmen, MR-Aufnahmen, Röntgenaufnahmen oder andere mittels medizinischer bildgebender System aufgenommene Bilder zum Beispiel durchschnittlicher Körper oder Körperteile beispielsweise einer bestimmten Personengruppe, wie Personen einer bestimmten Größe oder eines best immten Alters, verwendet werden. Diese Aufnahmen können in einer Datenbank gespeichert sein und zur Erstellung des generischen Modells abgerufen werden. Auch können bereits erstellte generische Modelle verwendet werden, welche beispielsweise auf Körperteilen oder Körperstrukturen eines bestimmten Personenkreises basieren. Insbesondere sind die generischen Modelle Oberflächenmodelle eines Körpers oder Körperteils.

Weiter werden zweidimensionale patientencharakteristische Erfassungsdaten eines Patienten beispielsweise dadurch ermittelt, dass mindestens zwei Fluoro-Bilder des Patienten aufgenommen oder bereitgestellt werden. Zum Beispiel können die Fluoro-Bilder des Patienten bei vorhergehenden Untersuchungen oder Eingriffen aufgenommen worden sein und in der Datenbank gespeichert worden sein.

Das patientenunspezifische dreidimensionale generische Modell bildet beispielsweise die typische Form und/oder Struktur eines Körpers oder Körperteils ab, welches auf Körperteilen oder Körperstrukturen einer Vielzahl von Personen basieren kann und somit nicht spezifisch für einen bestimmten Körper oder einen bestimmten Patienten ist. Die patientenspezifischen Fluoro-Aufnahmen enthalten patientenspezifische zweidimensionale Bilddaten, welche charakteristisch für einen bestimmten Körper oder Patienten sind.

Das generische Modell wird erfindungsgemäß an die zweidimensionalen patientenspezifischen Fluoro-Bilddaten oder patientencharakteristischen Erfassungsdaten angepasst. Dabei werden die Bilddaten oder Körperstrukturdaten des generischen Modells mittels einer Transformationsvorschrift an die zweidimensionalen patientencharakteristischen Erfassungsdaten angepasst. Mit anderen Worten werden die Bilddaten des generischen Modells verformt oder verändert, um eine Annäherung des dreidimensionalen generischen Modells oder einer zweidimensionalen Projektion des generischen Modells an die patientenspezifischen Erfassungsdaten zu gewährleisten. Eine Möglichkeit zur Anpassung des generischen Modells an die patientenspezifischen Erfassungsdaten, wie die zumindest zwei patientenspezifischen Fluoro-Bilddaten, wird in der Europäischen Patentanmeldung mit dem Titel "Shape reconstruction using x-ray images" und der Anmeldenummer 06012256.1 der Anmelderin beschrieben. Dabei werden ein allgemeines dreidimensionales Modell einer Struktur erhalten, mindestens zwei zweidimensionale Bilder der Struktur erhalten, zumindest ein Bildmerkmal in dem Bild bestimmt, die Orientierung des allgemeinen dreidimensionalen Modells der Struktur bezüglich der mindestens zwei zweidimensionalen Bilder der Struktur bestimmt, so dass das mindestens eine Bildmerkmal einer zweidimensionalen Projektion des dreidimensionalen Modells mit dem zweidimensionalen Bildmerkmal übereinstimmt oder zu diesem sehr ähnlich ist und das dreidimensionale Modell nach der Bestimmung der Orientierung des dreidimensionalen Modells gemorpht oder verformt wird, um die Form des dreidimensionalen Modells an die mindestens zwei zweidimensionalen Bilder anzupassen.

Erfindungsgemäß gibt es eine Korrelation zwischen dem generischen Modell und zumindest einem beispielsweise generischen oder durchschnittlichen oder typischen MR-Referenzdatensatz eines Körpers oder Körperteils oder einer Körperstruktur. Bevorzugt basiert das generische Modell zumindest auf diesem mindestens einen MR-Referenzdatensatz und kann insbesondere auch auf weiteren Referenzdatensätzen, wie CT-Referenzdatensätzen, basieren.

Durch das Anpassen des generischen Modells oder der zweidimensionalen Projektion des generischen Modells an die patientenspezifischen Fluoro-Bilddaten wird erfindungsgemäß eine Transformationsvorschrift erhalten, mittels welcher die Körperstrukturdaten des generischen Modells verändert oder verformt werden. Mittels dieser Transformationsvorschrift wird zumindest ein Teil des dreidimensionalen MR-Referenzdatensatzes verformt oder verändert. Damit wird der MR-Referenzdatensatz an die patienten- oder körperspezifischen Fluoro-Bilddaten angepasst. Somit wird aus dem MR-Referenzdatensatz ein an die Fluoro-Bilder angepasster und/oder ein mit den Fluoro-Bildern registrierter patientencharakteristischer dreidimensionale MR-Datensatz erzeugt oder ermittelt.

Weiter können sowohl das zumindest teilweise angepasste generische Modell als auch ein daraus erzeugter oder ein dazu korrespondierender zumindest teilweise patientencharakteristischer dreidimensionaler MR-Datensatz durch eine starre Transformation mit einem weiteren patientencharakteristischen dreidimensionalen MR-Datensatz verknüpft werden, um eine Navigation auf patientencharakteristischen dreidimensionalen MR-Daten zu ermöglichen.

Die Vorteile der vorliegenden Erfindung beruhen unter anderem darauf, dass es mit der Verwendung eines an den Patienten angepassten generischen oder statistischen Modells nicht mehr notwendig ist, für die Behandlung, bei der eine medizinische Navigation bereitgestellt werden soll, einen separaten Datensatz für die Körperstruktur zu erstellen. Zum einen erspart man dem Patienten dabei hohe Strahlungsbelastungen beispielsweise durch die Aufnahme zahlreicher Röntgen- oder CT-Bilder. Zum anderen können Kosten für solche Datensatzerstellungen eingespart werden, während andererseits durch die Verknüpfung der generischen Körperstrukturdaten mit patientencharakteristischen Erfassungsdaten ein Datensatz zur Verfügung gestellt wird, mit dessen Hilfe eine sehr genaue medizinische Navigation ermöglicht wird. Das generische Modell, das eine Art allgemeingültiges Modell für die betreffende Körperstruktur sein kann, für das sämtliche relevante Daten vorliegen, umfasst von vornherein zwar keine Daten, die speziell auf den betreffenden Patienten zugeschnitten sind, umfasst jedoch ausreichend Anatomie- oder Körperstrukturdaten, um nach der Anpassung mit Hilfe patientencharakteristischer Erfassungsdaten eine ausreichend genaue Basis für eine medizinische Navigation zur Verfügung stellen zu können.

Mit dem angepassten Bilddatensatz oder dem angepassten Modell kann ebenso gearbeitet werden, wie mit einem Bilddatensatz, der kostenintensiv und unter Strahlungsbelastung präoperativ von dem Patienten erstellt worden ist. Es ist beispielsweise denkbar, ein generisches Modell zu verwenden, das eine typische bzw. durchschnittliche Körperstruktur umfasst, beispielsweise eine einfache modellhafte Darstellung eines Wirbelkörpers, eines Oberarm-, Unterarm-, Oberschenkel-, Unterschenkel- oder Beckenknochens, oder einer sonstigen knöchernen Körperstruktur und/oder Weichteilstruktur.

Das generische Modell kann auch ein statistisches Modell der Körperstruktur umfassen, insbesondere basierend auf statistischen Auswertungen einer unbestimmten Anzahl von Bilddatensätzen, zum Beispiel von realen Wirbelkörper-Bilddatensätzen.

Ferner besteht die Möglichkeit, das generische Modell gleich als eine Art Modell-Paket für eine Vielzahl von Körperstrukturen gleicher Art bereitzustellen. In einem solchen Fall ist es bei der Anpassung des Modells möglich, sich aus der Vielzahl der Modelle im Paket dasjenige auszusuchen, welches am besten zu den patientencharakteristischen Erfassungsdaten passt, so dass nur eine geringere computergestützte Anpassung des Modells vorgenommen werden muss.

Das generische Modell kann im Rahmen der vorliegenden Erfindung einen zwei- oder dreidimensionalen Datensatz einer Körperstruktur umfassen, insbesondere auch ein geometrisches Modell. Mit anderen Worten kann es sich beim generischen Modell sowohl um dreidimensionale (zum Beispiel Wirbelkörpermodell) als auch um zweidimensionale Daten (zum Beispiel virtuelle Röntgenbilder) oder aber auch um ein Modell in Form von geometrischen Daten handeln. Diese Daten können beispielsweise Winkel und/oder Trajektorieninformationen sein, die für den Arzt dargestellt werden und ihm beispielsweise die Idealposition eines Implantates anzeigen.

Das generische Modell ist erfindungsgemäß mit mindestens einem MR-Referenzdatensatz korreliert, so dass Veränderungen oder Verformungen oder Anpassungen des generischen Modells mittels der Transformationsvorschrift auf den mindestens einen MR-Referenzdatensatz analog angewendet werden können, um den MR-Referenzdatensatz zur Erzeugung eines zumindest nahezu patientencharakteristischen MR-Datensatzes zu verändern.

Auch kann das generische Modell aus mindestens einem dreidimensionalen CT-Referenzdatensatz und dem mindestens einen dreidimensionalen MR-Referenzdatensatz gebildet werden, so dass der CT-Referenzdatensatz und der MR-Referenzdatensatz sozusagen zu dem generischen Modell korrespondierende dreidimensionale CT-Referenzdarstellungen oder CT-Referenzmodelle bzw. MR-Referenzdarstellungen oder MR-Referenzmodelle bilden können. Dabei sind oder werden die korrespondierenden CT- und MR-Referenzdatensätze vorzugsweise miteinander registriert.

Insbesondere kann das generische oder statistische Modell aus einer Vielzahl von CT-Referenzdatensätzen und einer Vielzahl von korrespondierenden MR-Datensätzen gebildet werden, welche miteinander registriert werden oder sein können.

Bevorzugt wird zur Bildung des generischen Modells eine Vielzahl an CT-Referenzdatensätzen ermittelt oder bereitgestellt. Weiter werden vorzugsweise zu der Vielzahl von CT-Referenzdatensätzen korrespondierende MR-Referenzdatensätze ermittelt oder bereitgestellt und die MR-Referenzdatensätze werden bevorzugt mit ihren korrespondierenden CT-Referenzdatensätzen registriert. Aus diesen miteinander registrierten Datensätzen kann das generische Modell, beispielsweise als Oberflächenmodell, erstellt werden, wobei aus der Vielzahl von CT-Referenzdatensätzen ein CT-Referenzmodell und aus der Vielzahl von MR-Referenzdatensätzen ein MR-Referenzmodell erstellt werden kann, welche mit dem generischen Modell korreliert sein können oder in eindeutiger Zuordnung stehen können oder welche dem generischen Modell unterliegen.

Wird das generische Modell durch Anpassung an die patientenspezifischen Bilddaten oder Erfassungsdaten verformt, so gehorcht diese Verformung einer Transformations- oder Abbildungsvorschrift. Durch die Verformung des generischen Modells kann ein an die realen Körperstrukturen des Patienten angepasstes Modell erstellt werden. Wendet man die Transformationsvorschrift auf die mit dem generischen Modell korrelierten MR-Referenzdatensätze oder auf das MR-Referenzmodell an, so ergibt sich eine an die Körperstrukturen des Patienten angenäherte MR-Darstellung. Damit ist vorzugsweise eine Beziehung zwischen der ermittelten MR-Darstellung und den Fluoro-Bildern des Patienten hergestellt, so dass die den realen Patientendaten angenäherten MR-Referenzdaten oder das angepasste MR-Referenzmodell mit den Fluoro-Bildern registriert sein können. Auch kann die Transformationsvorschrift auf das CT-Referenzmodell angewendet werden, um ein den realen Patientendaten angepasstes CT-Referenzmodell zu erhalten.

Zudem kann auch das angepasste generische Modell mittels einer festen Registrierung oder Transformation mit einem korrespondierenden patientencharakteristischen MR-Datensatz registriert werden. Somit kann das angepasste generische Modell weiter angepasst oder den realen Patientenstrukturen, insbesondere den realen Weichteilstrukturen angenähert werden. Auch kann nur das angepasste dreidimensionale MR-Referenzmodell mit dem patientencharakteristischen MR-Datensatz mittels einer festen Registrierung registriert werden, um ein genaueres oder patientenspezifischeres MR-Modell zu erhalten.

Auch kann eine Vielzahl an CT-Referenzdatensätzen erstellt oder bereitgestellt werden, aus denen das generische Modell erzeugt wird. Beispielsweise kann aus der Vielzahl von CT-Referenzdatensätzen ein CT-Referenzdatensatz als ein CT-Hauptform-Referenzdatensatz ausgewählt werden. Zu diesem CT-Hauptform-Referenzdatensatz kann ein korrespondierender MR-Hauptform-Referenzdatensatz erstellt oder bereitgestellt werden, wobei der CT-Hauptform-Referenzdatensatz mit dem MR-Hauptform-Referenzdatensatz registriert werden oder sein kann.

Das generische Modell kann somit ausschließlich aus oder mittels CT-Referenzdatensätzen erzeugt werden, so dass das generische Modell genau und schnell an die patientenspezifischen Fluoro-Bilder angepasst werden kann. Vorzugsweise kann das generische Modell aus der Vielzahl von CT-Referenzdatensätzen, wie CT-Trainings-Referenzdatensätzen und dem CT-Hauptform-Referenzdatensatz, erzeugt werden. Der eine MR-Hauptform-Referenzdatensatz ist oder wird vorzugsweise mit dem CT-Hauptform-Referenzdatensatz registriert. Bevorzugt besteht eine Korrelation zwischen dem generischen Modell und dem MR-Hauptform-Referenzdatensatz.

Beispielsweise kann eine Vielzahl an CT-Referenzdatensätzen des Rückenwirbels von verschiedenen Personen erstellt werden, wie zwei, drei, vier, fünf, sechs, sieben, acht oder mehr CT-Referenzdatensätze. Beispielhaft können sechs CT-Referenzdatensätze von Rückenwirbeln verschiedener Personen aus einer Datenbank ausgelesen oder ermittelt werden. Einer der sechs CT-Referenzdatensätze wird dabei vorzugsweise als CT-Hauptform-Referenzdatensatz ausgewählt. Weiter wird bevorzugt ein mit dem CT-Hauptform-Referenzdatensatz registrierter MR-Hauptform-Referenzdatensatz aus der Datenbank ausgelesen oder es wird ein zu dem CT-Hauptform-Referenzdatensatz korrespondierender MR-Hauptform-Referenzdatensatz erstellt, welcher mit dem CT-Hauptform-Referenzdatensatz registriert wird oder ist. Basierend auf den übrigen fünf CT-Referenzdatensätzen kann das generische Modell beispielsweise dadurch ermittelt werden, dass nur die extrahierte Oberflächeninformation der fünf CT-Referenzdatensätze verwendet wird, um ein Oberflächenmodell als generisches Modell zu erstellen. Eine Möglichkeit zur Erstellung des generischen Modells als Oberflächenmodell ist beschrieben in M. Fleute and S. Lavallee. Building a complete surface model from sparse data using statistical shape models: Application to computer assisted knee surgery. In MICCAI, pages 878-887, 1998.

Das generische Modell kann durch Verformung zumindest eines Teils des generischen Modells an die patientenspezifischen Fluoro-Bilder mittels einer Anpassungs- oder Transformationsvorschrift, wie einer dreidimensionalen Transformationsvorschrift, angepasst werden, welche beschreibt oder angibt, wie ein Teil oder das gesamte generische Modell verschoben oder umpositioniert werden müssen, um eine größtmögliche Übereinstimmung des generischen Modells mit den patientenspezifischen Bilddaten zu erreichen.

Diese Transformationsvorschrift kann beispielsweise auf den gesamten MR-Hauptform-Referenzdatensatz angewendet werden, um eine annähernd patientencharakteristische MR-Darstellung aus dem MR-Referenzdatensatz zu erhalten, welche in Beziehung zu den Fluroro-Bilddaten steht. Weiter kann die Transformationsvorschrift auch analog auf den CT-Hauptform Referenzdatensatz angewendet werden, um dessen Bilddaten zu transformieren oder zu verformen.

Auch kann die Transformationsvorschrift nur auf einen Teil des mit dem CT-Hauptform-Referenzdatensatz registrierten MR-Hauptform-Referenzdatensatzes oder einen aus dem CT-Hauptform-Referenzdatensatz und dem MR-Hauptform-Referenzdatensatzes gebildeten Hauptform-Referenzdatensatz angewendet werden. Vorzugsweise wird die Transformationsvorschrift nur auf die Bereiche oder Strukturen des Hauptform-Referenzdatensatz oder MR-Hauptform-Referenzdatensatzes angewendet, welche eindeutig in den Fluoro-Bilddaten und/oder dem generischen Modell erkennbar oder referenzierbar sind, so dass bezüglich dieser Punkte eine genaue oder zuverlässige Transformation gewährleistet werden kann. Die nicht in den Fluoro-Bildern und/oder dem generischen-Modell, wie dem aus den CT-Datensätzen generierten generischen Modell, dargestellten Strukturen, wie Weichteilstrukturen oder Muskeln oder Hautstrukturen, können unverändert bleiben, also nicht der Transformation unterworfen werden. Somit kann ein mit dem CT-Hauptform-Referenzdatensatz registrierter MR-Hauptform-Referenzdatensatz ermittelt werden oder ein aus dem CT-Hauptform-Referenzdatensatz und dem MR-Hauptform-Referenzdatensatz gebildeter Hauptform-Referenzdaten erzeugt werden, bei welchem lediglich die Strukuren oder Teile an die patientenspezifischen Bilddaten angepasst wurden, welche mit hoher Wahrscheinlichkeit angepasst oder verformt werden mussten. Die Bilddaten, bei welchen eine Unsicherheit besteht, ob eine Verformung nötig ist, wie Bilddaten, welche nicht in den Fluoro-Bildern und/oder dem generischen Modell erkannt oder dargestellt werden können, bleiben vorzugsweise unverändert.

Weiter kann der an die realen Patientenstrukturen angenäherte Hauptform-Referenzdatensatz oder MR-Hauptform-Referenzdatensatz dadurch verbessert oder weiter an die realen Patientenstrukturen angepasst werden, indem der bereits angepasste Hauptform-Referenzdatensatz oder MR-Hauptform-Referenzdatensatz mittels einer festen Registrierung oder Transformation mit einem korrespondierenden patientencharakteristischen MR-Datensatz registriert wird. Dadurch können die Bereiche des angepassten MR-Hauptform-Referenzdatensatzes ausgefüllt oder verändert werden, welche zunächst unverändert geblieben sind.

Diese Vorgehensweise weist eine Reihe von Vorteilen auf. Es können einfach zu ermitteltende oder aufzunehmende patientenunspezifische CT-Datensätze verwendet werden, um das generische Modell zu erstellen. Lediglich der Hauptform-Referenzdatensatz umfasst bei dieser Vorgehensweise einen MR-Hauptform-Referenzdatensatz und einen CT-Hauptform-Referenzdatensatz, was zu einer erheblichen Aufwands- und Kostenreduzierung führt. Auch werden nur die Daten des Hauptform-Referenzdatensatzes basierend auf der ermittelten Transformationsvorschrift verändert, die auch in den Fluoro-Bildern und/oder dem generischen Modell darstellbar sind und deren patientenspezifische Position somit mit hoher Wahrscheinlichkeit korrekt ermittelt werden kann. Auch die feste Registrierung des angepassten Hauptform-Referenzdatensatzes mit dem patientenspezifischen MR-Datensatz stellt einen einfachen, genauen und schnellen Vorgang dar.

Im Folgenden werden verschiedene Arten von patientencharakteristischen Daten aufgezeigt, wie sie zur Anpassung des generischen Modells verwendet werden können. Es ist immer auch möglich, Kombinationen solcher Daten, die im Weiteren als Diagnosedaten bezeichnet werden, hierfür einzusetzen.
Die patientencharakteristischen Daten können Röntgenbilddaten sein, und zwar solche aus vorab oder während der Behandlung erstellten Röntgenbildern, insbesondere zwei- oder multiplanaren Röntgenbildern. Ein Beispielsfall wäre derjenige, wo für den Patienten schon Röntgenbilder vorliegen, die im Rahmen zeitlich zurückliegender Untersuchungen erstellt worden sind. Daten über Körperstrukturen aus diesen "alten" Röntgenbildern eignen sich insbesondere, wenn Formabweichungen gegenüber dem generischen Modell einberechnet werden sollen.

Es ist aber auch möglich, noch während der Behandlung einzelne Röntgenbilder des Patienten zu erstellen und diese Informationen dann in die Anpassung des generischen Modells einfließen zu lassen. Der Vorteil gegenüber der herkömmlichen "Röntgennavigation" liegt dabei darin, dass nicht eine große Vielzahl von Röntgenbildern erstellt werden muss, wie sie bei der Navigation auf der Basis von Röntgenbildern verwendet wird. Hingegen genügt die Erstellung nur eines oder sehr weniger Röntgenbilder zur Anpassung des generischen Modells, die sich außerdem auf einen sehr kleinen Körperabschnitt begrenzen können. Damit wird die Strahlenbelastung gegenüber herkömmlicher Röntgennavigation stark verringert.

Obiges gilt in gleicher Weise auch für Computertomographie- oder Kemspintomographie-Bilddaten. Es können solche verwendet werden, die aus sehr viel früher erstellten Tomographieerfassungen hervorgehen, deren Information jedoch genügt, um eine geeignete Anpassung des generischen Modells vorzunehmen.

Es ist aber nicht unbedingt nötig, in dieser Weise komplizierte, patientencharakteristische Erfassungsdaten bzw. Diagnosedaten zu verwenden, um eine ausreichende Anpassung des generischen Modells vornehmen zu können. Es kann durchaus genügen, akquirierte Punkt-Positionsinformationen der Patienten-Körperstruktur, insbesondere von natürlichen oder künstlichen Landmarken, zu benutzen. Bei den patientencharakteristischen Diagnosedaten kann es sich dann beispielsweise nur um den Abstand zwischen zwei Landmarken (zum Beispiel Knochenfortsätzen) handeln, welcher schon genügend Informationen darüber geben kann, in welcher Weise das generische Modell zu restrukturieren ist. Ebenso können als Basis hierfür Daten über Größe, Gewicht oder Körperabschnitts- bzw. Gliedmassenlängen des Patienten zum Einsatz kommen.

Die Anpassung des generischen Modells kann im Rahmen der Erfindung durch eine oder mehrere der folgenden Methoden erfolgen:
manuelle Anpassung mit Hilfe einer Bilddarstellungsunterstützung, insbesondere durch das Versetzen von Punkten und Landmarken oder das Verschieben, Verdrehen, Dehnen, oder Komprimieren des generischen Modells auf einer Bildschirmausgabe mittels Benutzerinterface-Einrichtungen, automatische Bildfusionsverfahren, die insbesondere auf der automatischen Erkennung bestimmter anatomischer Merkmale basieren, Bilddaten des generischen Modells, insbesondere digital rekonstruierte Röntgenbilder, und solche aus Computertomographie- oder Kemspintomographie-Bilddatensätzen werden in Deckung gebracht bzw. fusioniert.

Insbesondere kann als die Transformationsvorschrift des generischen Modells eine Verformungs- und/oder eine Rotationsvorschrift verwendet wird.

Die Fusion des generischen Modells mit patientenspezifischen Informationen oder Bilddaten kann also entweder automatisch erfolgen, zum Beispiel durch automatische Erkennung bestimmter anatomischer Merkmale, die für die Fusion entscheidend sind, oder aber manuell, zum Beispiel durch Verschieben, Verdrehen, Stretchen/Verziehen. Wenn eine Fusion des generischen Modells mit echten Patienteninformationen mit Hilfe der Akquirierung einer unbestimmten Anzahl von Punktinformationen am Patienten erfolgt (Landmarken), ist es möglich, ein sogenanntes Surface-Matching-Verfahren, also ein computergestütztes Bildanpassungsverfahren zu verwenden, um die Fusion der Bilddaten durchzuführen. Eine Kombination der Diagnosedatenerfassung sowie der Anpassung des generischen Modells aus verschiedenen oben beschriebenen Methoden kann so durchgeführt werden, dass neben den diagnostischen Daten (zum Beispiel intraoperativ akquirierte Röntgenbilder) noch zusätzliche Punkte am Patienten in Form von Landmarken oder zufällig akquirierten Punkten aufgenommen und dazu verwendet werden, die Position des Modells oder dessen Form selbst noch genauer zu erfassen und einzurichten, um damit eine genauere Navigation zu ermöglichen.

Allgemein gesagt, können bei dem erfindungsgemäßen Verfahren die Positionsdaten, die bei der Ermittlung der patientencharakteristischen Erfassungsdaten erhalten werden, insbesondere durch die Akquirierung von Landmarkenpositionen oder durch in einem Navigationssystem registrierte Röntgenbildaufnahmen, dazu verwendet werden, die Registrierung der angepassten Körperstrukturdaten im Navigationssystem vorzunehmen und Behandlungsgeräte bzw. behandlungsunterstützende Geräte in Registrierung zur angepassten Körperstruktur bildlich darzustellen oder einzusetzen. Mit anderen Worten wird der Schritt der Erfassung der Diagnosedaten dabei auch gleichzeitig dazu genutzt, die Registrierung von Patient und angepasstem generischen Modell für die Navigation vorzunehmen. Sobald die Daten des Modells mit registrierten Daten, d. h. Daten, die im Raum eindeutig in ihrer Position bestimmt sind, zum Beispiel registrierten Fluoroskopiebildern einer Röntgennavigationssoftware fusioniert werden, oder die Daten des Modells mit Landmarken oder einer Kombination beider Verfahren registriert werden, können diese für die computerassistierte Chirurgie und zum Beispiel für minimal invasive Operationen eingesetzt werden, in dem Instrumente oder Implantate in Relation zu einem fusionierten Modell dargestellt werden.

Die Erfindung bezieht sich des Weiteren auf ein Computerprogramm, welches, wenn es in einen Computer geladen wird oder auf einem Computer läuft, ein wie oben beschriebenes Verfahren durchführt. Weiterhin bezieht sich die Erfindung auf ein Programmspeichermedium oder ein Computerprogrammprodukt mit einem solchen Programm.

Die erfindungsgemäße Vorrichtung zur Registrierung eines dreidimensionalen Magnetresonanz(MR)-Datensatzes mit mindestens zwei Fluoro-Bildern eines Patienten, umfasst eine Datenerfassungsvorrichtung, ein Navigationssystem zum Ermitteln der dreidimensionalen Raumposition der Datenerfassungsvorrichtung und eine mit dem Navigationssystem verbundenen Recheneinheit, wie einen Computer. Mittels der Datenerfassungsvorrichtung können patientenspezifische Erfassungsdaten, wie Fluoro-Bilder, aufgenommen werden. Dabei dient das Navigationssystem dazu die dreidimensionale Raumposition der Datenerfassungsvorrichtung relativ zu der aufzunehmenden Körperstruktur zu ermitteln.

Mit dem Navigationssystem ist die Recheneinheit vorzugsweise drahtlos oder drahtgebunden verbundenen. Mit der Recheneinheit können alle zuvor beschriebenen Verfahrensschritte ausgeführt oder ermittelt werden, welche Rechenoperationen verlangen. Die Recheneinheit kann ein dreidimensionales generisches Modell, insbesondere ein Oberflächenmodell für Körper oder Körperteile verschiedener Personen ermitteln, wobei das generische Modell patientenunspezifische Körperstrukturdaten enthält. Das dreidimensionale generische Modell kann zumindest einen dreidimensionalen MR-Referenzdatensatz enthalten oder auf diesem basieren oder kann zumindest mit dem dreidimensionalen MR-Referenzdatensatz korreliert sein. Die Recheneinheit kann das generische Modell basierend auf den ermittelten zweidimensionalen patientencharakteristischen Erfassungsdaten mittels einer Transformationsvorschrift zur Datenverknüpfung der Körperstrukturdaten des dreidimensionalen generischen Modells mit den zweidimensionalen patientencharakteristischen Erfassungsdaten verändern oder verformen oder anpassen. Weiter kann die Recheneinheit zumindest einen Teils des dreidimensionalen MR-Referenzdatensatzes mittels der Transformationsvorschrift verändern oder anpassen, um einen mit den Fluoro-Bildern registrierten patientencharakteristischen dreidimensionalen MR-Datensatz zu erzeugen oder zu ermitteln.

Die Erfindung wird nachfolgend anhand bevorzugter Ausführungsbeispiele beschrieben werden. Es zeigen:
- Figur 1: ein Ablaufdiagram eines beispielhaften Verfahrens
- Figur 2: ein Ablaufdiagram gemäß einer zweiten Ausführungsform des erfindungsgemäßen Verfahrens
- Figur 3: eine Ausführungsform einer erfindungsgemäßen Vorrichtung

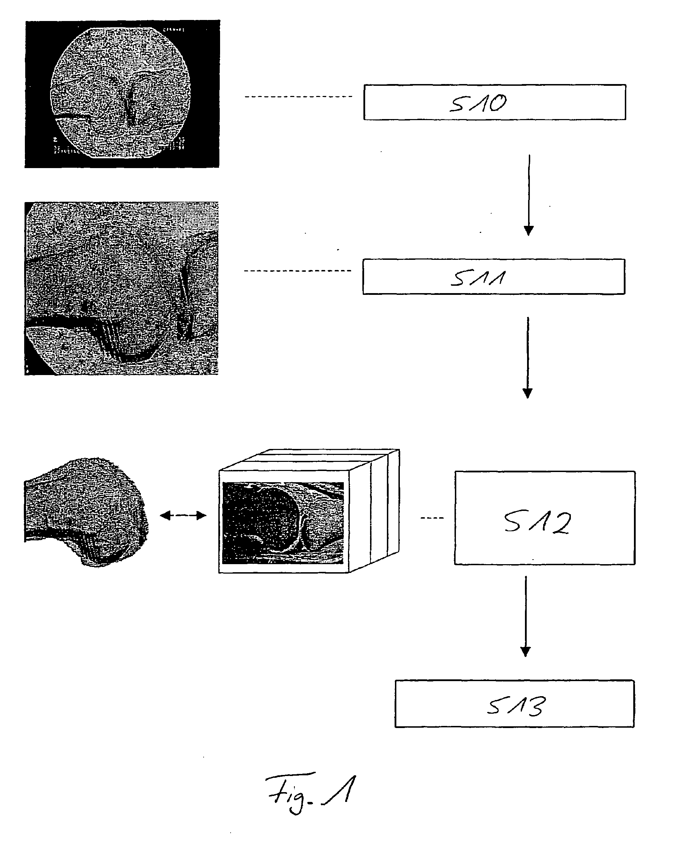

Figur 1 zeigt ein Ablaufdiagram eines beispielhaften Verfahrens. Zunächst werden in Schritt S10 mindestens zwei Fluoro-Bilder eines Körperbereichs eines Patienten aufgenommen. Diese Fluoro-Bilder enthalten patientenspezifische Daten, wie patientenspezifische Strukturen oder Formen. Ein patientenunspezifisches adaptives generisches Modell, welches beispielsweise aus einer Vielzahl an Datensätzen, wie CT-, MR-, Röntgen- oder weiten Datensätzen bestehen kann, wird in Schritt S11 mittels einer Transformationsvorschrift an die Fluoro-Bilddaten angepasst, so dass das zunächst patientenunspezifische generische Modell zumindest teilweise an die realen patientenspezifischen aus den Fluoro-Bildern ersichtlichen Strukturen angepasst wird. In einem nächsten Schritt S 12 wird das bereits teilweise angepasste generische Modell bezüglich eines patientenspezifischen MR-Datensatzes registriert oder umgekehrt wird der patientenspezifische MR-Datensatz bezüglich des bereits teilweise angepassten generischen Modells registriert, wobei vorzugsweise eine starre Registrierung verwendet wird, welches das generische Modell nicht deformiert und beispielsweise bislang unbestimmte Strukturen oder Bereiche ausfüllen kann. Damit ist auch ein in dem generischen Modell enthaltener MR-Referenzdatensatz mit den patientenspezifischen Bilddaten registriert. In einem weiteren Schritt S 13 kann eine Navigation basierend auf den Fluoro-Bildern, dem generischen Modell und dem patientenspezifischen MR-Datensatz erfolgen.

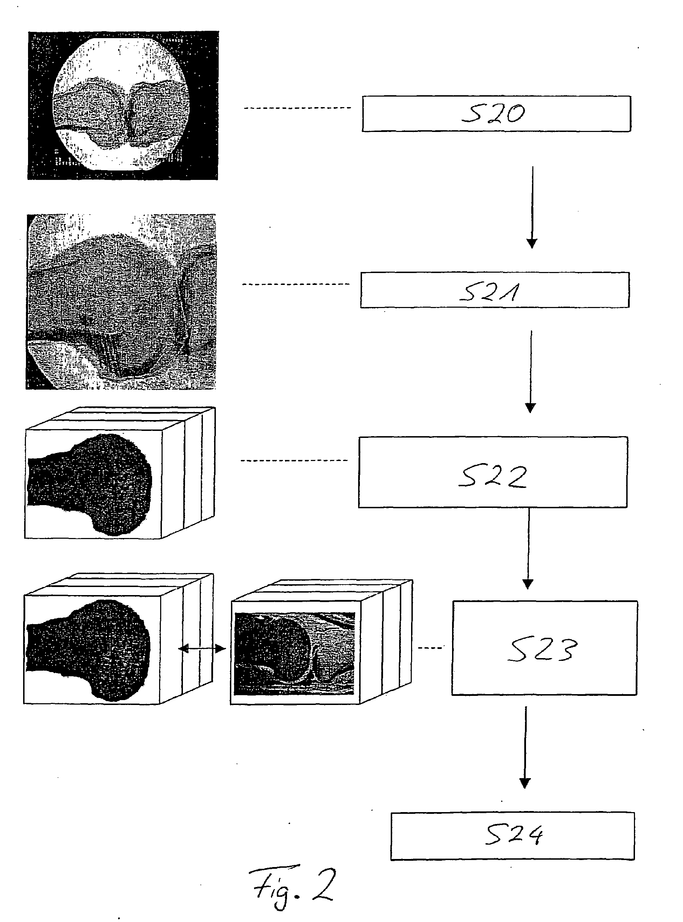

Figur 2 zeigt ein Ablaufdiagram einer zweiten Ausführungsform des erfindungsgemäßen Verfahrens. Wie in Bezug auf Figur 1 erläutert werden in Schritt S20 Fluoro-Bilder eines Patienten erstellt. Weiter wird ein körperunspezifisches generisches Modell ermittelt, welches auf CT-und MR-Datensätzen einer Vielzahl von Personen basiert. Dieses generische Modell wird in Schritt S21 mittels einer Transformation an die Fluoro-Bilder angepasst, so dass die Bereiche des generischen Modells verformt werden, welche in den körperspezifischen Fluoro-Bildern anders ausgebildet oder geformt sind. Damit wird in Schritt S21 eine Transformationsvorschrift ermittelt, welche die Verformung des körperunspezifischen generischen Modells zur Anpassung an die körperspezifische Bilddaten beschreibt. Mittels dieser Transformationsvorschrift wird ein mit dem generischen Modell in Beziehung stehender MR-Referenz-Datensatz in Schritt S22 verformt, wobei vorzugsweise nur die Bereiche oder Strukturen des MR-Referenz-Datensatzes verformt werden, welche auch in dem generischen Modell ausgebildet sind oder dargestellt sind. Basierend auf dem teilweise angepassten MR-Referenz-Datensatz kann bereits eine Navigation erfolgen. Optional wird zusätzlich der bereits teilweise angepasste MR-Referenz-Datensatz in Schritt S23 mit einem patientenspezifischen ermittelten MR-Datensatz vorzugsweise starr oder fest registriert oder umgekehrt wird der patientenspezifische MR-Datensatz mit dem bereits teilweise angepassten MR-Referenz-Datensatz vorzugsweise starr registriert, so dass bevorzugt zwar bislang unveränderte Bereiche oder im Vergleich mit dem MR-Datensatz unterschiedliche Bereiche des MR-Referenz-Datensatzes ausgefüllt oder verändert werden, jedoch keine Deformation oder Verformung des MR-Referenz-Datensatzes stattfindet. Somit ist der MR-Referenz-Datensatz mit den patientenspezifischen Daten, wie den Fluoro-Bilddaten registriert. In einem weiteren Schritt S24 kann eine Navigation basierend auf den Fluoro-Bildern, dem generischen Modell und dem patientenspezifischen MR-Datensatz erfolgen.

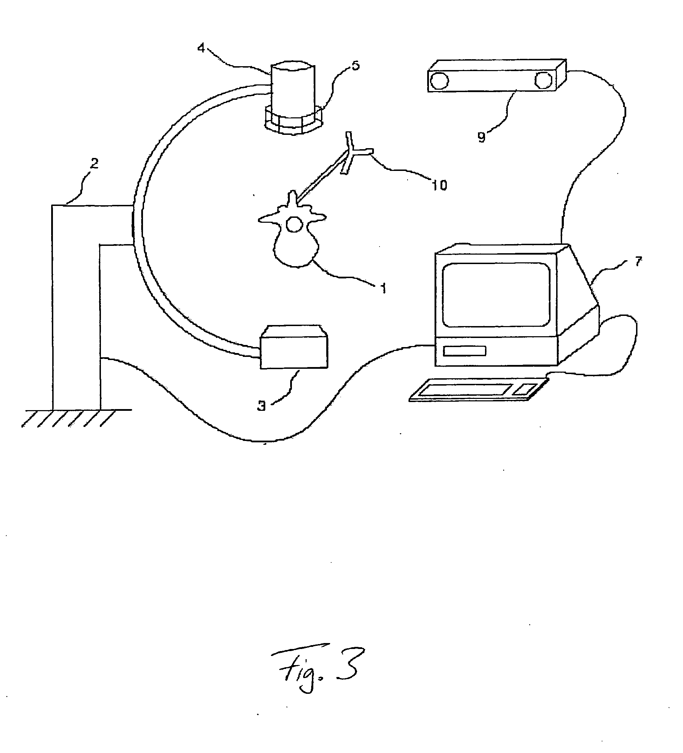

Figur 3 zeigt eine Ausführungsform der erfindungsgemäßen Vorrichtung, welche eine als C-Arm ausgebildete Röntgenvorrichtung 2 umfasst, mittels welcher Fluoro-Bilder eines Patienten, an welchem vorzugsweise ein Referenzstern 10 angeordnet ist, wie Aufnahmen des Rückenwirbels 1 eines Patienten, aufgenommen werden können. Die Röntgenvorrichtung weist eine Strahlungsquelle 3, einen Bildverstärker 4 und einen Röntgenschirm oder eine Röntgen-Ausstattung 5 oder auf, mittels welcher registrierte Fluoro-Bilder erstellt werden können. Weiter umfasst die Vorrichtung ein Navigationssystem 9, mittels dessen die Position der Röntgenvorrichtung bezüglich des Patienten, über die Erfassung des an dem Patienten angeordneten Referenzstems 10, ermittelt werden kann. Das Navigationssystem 9 ist mit einem Computer 7 verbunden, welcher einen Speicher oder eine Datenbank aufweist und welcher Rechenoperationen ausführen kann, die zur Durchführung des erfindungsgemäßen Verfahrens benötigt werden.

## Patentansprüche

1. Verfahren zur Registrierung eines dreidimensionalen Magnetresonanz (MR)-Referenzdatensatzes mit mindestens zwei Fluoro-Bildern eines Patienten, mit den folgenden Schritten:
- Erstellen oder Bereitstellen eines dreidimensionalen generischen Modells, insbesondere Oberflächenmodells, eines Körpers oder Körperteils, welches Körperstrukturdaten enthält wobei das generische Modell aus mindestens einem Computertomographie-Referendatensatz und dem mindestens einen MR-Referenzdatensatz gebildet wird, welche miteinander registriert werden oder sind;
- Ermitteln oder Bereitstellen von mindestens zwei Fluoro-Bildern als patientencharakteristischen zweidimensionalen Erfassungsdaten eines Patienten;
- Verändern oder Anpassen des dreidimensionalen generischen Modells des Körpers oder Körperteils basierend auf den ermittelten zweidimensionalen patientencharakteristischen Erfassungsdaten mittels einer Transformationsvorschrift zur Datenverknüpfüng der Körperstrukturdaten des dreidimensionalen generischen Modells mit den zweidimensionalen patientencharakteristischen Erfassungsdaten;
wobei das dreidimensionale generische-Modell zumindest mit dem dreidimensionalen MR-Referenzdatensatz korreliert ist und das Verfahren weiter den Schritt umfasst:
- Verändern oder Verformen zumindest eines Teils des dreidimensionalen MR-Referenzdatensatzes mittels der Transformationsvorschrift zum Erzeugen eines mit den Fluoro-Bildern registrierten patientencharakteristischen dreidimensionalen MR-Datensatzes.

2. Verfahren nach Anspruch, 1 wobei als die Transformationsvorschrift des generischen Modells eine Verformungs- und/oder eine Rotationsvorschrift verwendet wird.

3. Verfahren nach einem der vorhergehenden Ansprüche, wobei das generische Modell eines oder mehrere der folgenden Merkmale aufweist:
- es umfasst eine typische und/oder durchschnittliche Körperstruktur;
- es umfasst ein statistisches Modell einer Körperstruktur, insbesondere basierend auf statistischen Auswertungen einer unbestimmten Anzahl von Bilddatensätzen;
- es umfasst eine Vielzahl von Körperstrukturen gleicher Art;
- es umfasst einen zwei- oder dreidimensionalen Datensatz einer Körperstruktur, insbesondere auch ein geometrisches Modell.

4. Verfahren nach einem der vorhergehenden Ansprüche, wobei die Anpassung des generischen Modells durch eine oder mehrere der folgenden Methoden erfolgt:
- manuelle Anpassung mit Hilfe einer Bilddarstellungsunterstützung, insbesondere durch das Versetzen von Punkten und Landmarken oder das Verschieben, Verdrehen, Dehnen, oder Komprimieren des generischen Modells auf einer Bildschirmausgabe mittels Benutzerinterface-Einrichtungen,
- automatische Bildfusionsverfahren, die insbesondere auf der automatischen Erkennung bestimmter anatomischer Merkmale basieren,
- Bilddaten des generischen Modells, insbesondere digital rekonstruierte Röntgenbilder, und solche aus CT- oder MR-Bilddatensätzen werden in Deckung gebracht bzw. fusioniert,
worauf die angepassten Körperstrukturdaten computergestützt errechnet werden.

5. Computerprogramm, welches, wenn es auf einem Computer läuft, das Verfahren nach einem der vorhergehenden Ansprüche durchführt.

6. Programmspeichermedium oder Computerprogrammprodukt mit dem Computerprogramm nach dem vorhergehenden Anspruch.

7. Vorrichtung zur Registrierung eines dreidimensionalen Magnetresonanz (MR)-Referenzdatensatzes mit mindestens zwei Fluoro-Bildern eines Patienten, mit einer Datenerfassungsvorrichtung zum Aufnehmen der mindestens zwei Fluoro-Bilder, welche zweidimensionale patientencharakteristische Erfassungsdaten enthalten, mit einem Navigationssystem zum Ermitteln der dreidimensionalen Raumposition der Datenerfassungsvorrichtung relativ zu einem aufzunehmenden Körper oder Körperteil und mit einer mit dem Navigationssystem verbundenen Recheneinheit zum:
- Erstellen eines dreidimensionalen generischen Modells, insbesondere eines Oberflächenmodells, eines Körpers oder Körperteils, welches Körperstrukturdaten enthält, wobei das generische Modell aus mindestens einem Computertomographie-Referenzdatensatz und dem mindestens einen MR-Referendatensatz gebildet wird, welche miteinander registriert werden oder sind, und wobei das dreidimensionale generische Modell zumindest mit dem dreidimensionalen MR-Referenzdatensatz korreliert ist;
- Verändern oder Anpassen des generischen Modells des Körpers oder Körperteils basierend auf den ermittelten zweidimensionalen patientencharakteristischen Erfassungsdaten mittels einer Transformationsvorschrift zur Datenverknüpfung der Körperstrukturdaten des dreidimensionalen generischen Modells mit den zweidimensionalen patientencharakteristischen Erfassungsdaten; und
- Verändern oder Verformen zumindest eines Teils des dreidimensionalen MR-Referenzdatensatzes mittels der Transformationsvorschrift zum Erzeugen eines mit den Fluoro-Bildern registrierten patientencharakteristischen dreidimensionalen MR-Datensatzes.

## Claims

1. A method for registering a three-dimensional magnetic resonance (MR) reference data set to at least two fluoroscopic images of a patient, comprising the steps of:
- producing or providing a three-dimensional generic model, in particular a surface model, of a body or body part containing body structure data, wherein the generic model is formed from at least one computer tomography reference data set and the at least one MR reference data set, which are registered to each other;
- ascertaining or providing at least two fluoroscopic images as two-dimensional patient-characteristic detection data of a patient;
- changing or adapting the three-dimensional generic model of the body or body part, based on the ascertained two-dimensional patient-characteristic detection data, by means of a transformation protocol for data-linking the body structure data of the three-dimensional generic model to the two-dimensional patient-characteristic detection data;
wherein the three-dimensional generic model is at least correlated with the three-dimensional MR reference data set, and the method also comprises the step of:
- changing or deforming at least a part of the three-dimensional MR reference data set by means of the transformation protocol, in order to generate a patient-characteristic three-dimensional MR data set which is registered to the fluoroscopic images.

2. The method according to claim 1, wherein a deformation and/or rotation protocol is used as the transformation protocol of the generic model.

3. The method according to any one of the preceding claims, wherein the generic model exhibits one or more of the following features:
- it comprises a typical and/or average body structure;
- it comprises a statistical model of a body structure, in particular based on statistical evaluations of an indefinite number of image data sets;
- it comprises a plurality of body structures of the same type;
- it comprises a two-dimensional or three-dimensional data set of a body structure, and in particular a geometric model.

4. The method according to any one of the preceding claims, wherein the generic model is adapted by one or more of the following methods:
- manually adapting with the aid of image representation assistance, in particular by displacing points and landmarks or shifting, rotating, expanding or compressing the generic model on a screen output by means of user interface means;
- automatic image fusion methods which are in particular based on automatically identifying particular anatomical features;
- image data of the generic model, in particular digitally reconstructed x-ray images, and the same from CT or MR image data sets are superimposed or fused;
whereupon the adapted body structure data are calculated with computer assistance.

5. A computer program which, when it is running on a computer, performs the method according to any one of the preceding claims.

6. A program storage medium or computer program product comprising the computer program according to the preceding claim.

7. A device for registering a three-dimensional magnetic resonance (MR) reference data set to at least two fluoroscopic images of a patient, comprising: a data detection device for recording the at least two fluoroscopic images containing two-dimensional patient-characteristic detection data; a navigation system for ascertaining the three-dimensional spatial position of the data detection device relative to a body or body part to be recorded; and a computational unit connected to the navigation system, for:
- producing a three-dimensional generic model, in particular a surface model, of a body or body part containing body structure data, wherein the generic model is formed from at least one computer tomography reference data set and the at least one MR reference data set, which are registered to each other, and wherein the three-dimensional generic model is at least correlated with the three-dimensional MR reference data set;
- changing or adapting the generic model of the body or body part, based on the ascertained two-dimensional patient-characteristic detection data, by means of a transformation protocol for data-linking the body structure data of the three-dimensional generic model to the two-dimensional patient-characteristic detection data; and
- changing or deforming at least a part of the three-dimensional MR reference data set by means of the transformation protocol, in order to generate a patient-characteristic three-dimensional MR data set which is registered to the fluoroscopic images.

## Revendications

1. Procédé de recalage d'un jeu de données de référence tridimensionnelles de résonance magnétique (MR) avec au moins deux images fluoroscopiques d'un patient, avec les étapes suivantes:
- création ou mise à disposition d'un modèle générique tridimensionnel, en particulier d'un modèle de surface, d'un corps ou d'une partie du corps, qui contient des données de structure du corps, le modèle générique étant constitué d'au moins un jeu de données de référence de tomographie assistée par ordinateur et d'au moins un jeu de données de référence de résonance magnétique qui sont ou seront recalés l'un avec l'autre;
- détermination ou mise à disposition d'au moins deux images fluoroscopiques d'un patient comme données d'acquisition bidimensionnelles caractéristiques du patient;
- modification ou adaptation du modèle générique tridimensionnel du corps ou d'une partie du corps sur base des données d'acquisition bidimensionnelles déterminées caractéristiques du patient à l'aide d'une prescription de transformation pour l'interconnexion des données de structure du corps du modèle générique tridimensionnel avec les données d'acquisition bidimensionnelles caractéristiques du patient;
le modèle générique tridimensionnel étant corrélé au moins avec le jeu de données de référence tridimensionnelles de résonance magnétique et le procédé comprenant en outre l'étape de:
- modification ou déformation d'au moins une partie du jeu de données de référence tridimensionnelles de résonance magnétique à l'aide d'une prescription de transformation pour la génération d'un jeu de données tridimensionnelles de résonance magnétique caractéristiques du patient recalé avec les images fluoroscopiques.

2. Procédé suivant la revendication 1, une prescription de déformation et/ou de rotation étant utilisée comme prescription de transformation du modèle générique.

3. Procédé suivant l'une des revendications précédentes, le modèle générique présentant une ou plusieurs des caractéristiques suivantes:
- il comprend une structure du corps typique et/ou moyenne;
- il comprend un modèle statistique d'une structure du corps, en particulier sur base de l'exploitation statistique d'un nombre indéterminé de jeux de données d'image;
- il comprend un grand nombre de structures du corps de même type;
- il comprend un jeu de données bidimensionnel ou tridimensionnel d'une structure du corps, en particulier également un modèle géométrique.

4. Procédé suivant l'une des revendications précédentes, l'adaptation du modèle générique se faisant à l'aide d'un ou plusieurs des procédés suivants:
- adaptation manuelle à l'aide d'une assistance de visualisation, en particulier par le décalage de points et de points de repère ou par le décalage, la rotation, la dilatation ou la compression du modèle générique sur une sortie sur écran à l'aide de dispositifs d'interface utilisateur,
- procédés automatiques de fusion d'images, qui sont en particulier basés sur la détection automatique de caractéristiques anatomiques déterminées,
- des données d'image du modèle générique, en particulier des images radiographiques reconstruites numériquement, et des données d'image de jeux de données d'image de tomographie assistée par ordinateur ou de résonance magnétique sont mises en superposition ou fusionnées,
les données de structure du corps adaptées pouvant être ensuite être calculées par ordinateur.

5. Programme d'ordinateur qui, lorsqu'il tourne sur un ordinateur, exécute le procédé suivant l'une des revendications précédentes.

6. Support d'information pour programme ou produit de programme d'ordinateur comportant le programme d'ordinateur suivant la revendication précédente.

7. Dispositif de recalage d'un jeu de données de référence tridimensionnelles de résonance magnétique (MR) avec au moins deux images fluoroscopiques d'un patient, avec un dispositif d'acquisition de données pour l'enregistrement des au moins deux images fluoroscopiques qui contiennent des données d'acquisition bidimensionnelles caractéristiques du patient avec un système de navigation pour la détermination de la position spatiale tridimensionnelle du dispositif d'acquisition de données relativement à un corps ou une partie du corps à enregistrer et avec une unité de calcul liée au système de navigation pour:
- la création ou la mise à disposition d'un modèle générique tridimensionnel, en particulier d'un modèle de surface, d'un corps ou d'une partie du corps, qui contient des données de structure du corps, le modèle générique étant constitué d'au moins un jeu de données de référence de tomographie assistée par ordinateur et de l'au moins un jeu de données de référence de résonance magnétique qui sont ou seront recalés l'un avec l'autre, et le modèle générique tridimensionnel étant au moins corrélé avec le jeu de données de référence tridimensionnelles de résonance magnétique;
- la modification ou l'adaptation du modèle générique du corps ou d'une partie du corps sur base des données d'acquisition bidimensionnelles déterminées caractéristiques du patient à l'aide d'une prescription de transformation pour l'interconnexion des données de structure du corps du modèle générique tridimensionnel avec les données d'acquisition bidimensionnelles caractéristiques du patient; et
- la modification ou la déformation d'au moins une partie du jeu de données de référence tridimensionnelles de résonance magnétique à l'aide d'une prescription de transformation pour la génération d'un jeu de données tridimensionnelles de résonance magnétique caractéristiques du patient recalé avec les images fluoroscopiques.
